# EUROPEAN PATENT SPECIFICATION

(11) **EP 0 828 840 B1**
(45) Date of publication and mention of the grant of the patent: **10.01.2001**
(21) Application number: 96913073.1
(22) Date of filing: 26.04.1996
(51) Int. Cl.: C12N 15/56, C12N 15/63, C12N 9/42, C12N 1/21, C11D 3/386, D06M 16/00

(54) **ALKALINE CELLULASE AND METHOD FOR PRODUCING THE SAME**
ALKALISCHE CELLULASE UND VERFAHREN ZU DEREN HERSTELLUNG
CELLULASE ALCALINE ET SON PROCEDE DE FABRICATION

(30) Priority: 28.04.1995 EP 95201115
(43) Date of publication of application: 18.03.1998
(73) Proprietor: GENENCOR INTERNATIONAL, INC., Rochester, New York 14618 (US)
(72) Inventor: VAN SOLINGEN, Pieter, NL-2671 VZ Naaldwijk (NL)
(74) Representative: Kiddle, Simon John
(86) International application number: PCT/US96/05652
(87) International publication number: WO 96/34108

(56) References cited:
- WO-A-94/01532
- WO-A-95/18219
- JP-A- 62 269 692
- JP-A- 62 296 874
- Embl Database Entry BSCELB1; Accession number Z33876; 23rd May 1994; SANCHEZ TORRES J.: "Bacillus sp. celB1 gene for endo-beta-1,4-glucanase" XP002013980
- JOURNAL OF BACTERIOLOGY, vol. 168, no. 2, November 1986, pages 479-485, XP000600247 FUMIYASU FUKUMORI ET AL.: "Nucleotide sequences of two cellulase genes from alkalophikic Bacillus sp. strain N-4 and their strong homology" cited in the application
- PREBEN NIELSEN ET AL.:"Comparative 16S rDNA sequence analysis of some alkaliphilic bacilli and the establishment of a sixth rRNA group within the genus Bacillus, FEMS MICROBIOLOGY LETTERS, 1994, vol. 117, pages 61 to 66
- PREBEN NIELSEN ET AL.:"Phenetic diversity of alkaliphilic Bacillus strains: proposal for nine new species", MICROBIOLOGY, 1995, vol. 141, pages 1745 to 1761

### SEQUENCE LISTING

### (1) GENERAL INFORMATION:

(i) APPLICANT:
   (A) NAME: Gist-brocades
   (B) STREET: Wateringseweg 1
   (C) CITY: Delft
   (E) COUNTRY: The Netherlands
   (F) POSTAL CODE (ZIP) : 2611 XT
(ii) TITLE OF INVENTION: Novel Cellulase and Its Applications
(iii) NUMBER OF SEQUENCES: 2
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)

### (2) INFORMATION FOR SEQ ID NO: 1:

(i) SEQUENCE CHARACTERISTICS:
   (A) LENGTH: 1404 base pairs
   (B) TYPE: nucleic acid
   (C) STRANDEDNESS: double
   (D) TOPOLOGY: linear
(ii) MOLECULE TYPE: DNA (genomic)
(iii) HYPOTHETICAL: NO
(iii) ANTI-SENSE: NO
(vi) ORIGINAL SOURCE:
   (A) ORGANISM: Bacillus sp.
   (C) INDIVIDUAL ISOLATE: CBS 670.93
(ix) FEATURE:
   (A) NAME/KEY: sig_peptide
   (B) LOCATION: 1..78
(ix) FEATURE:
   (A) NAME/KEY: mat_peptide
   (B) LOCATION: 79..1404
   (D) OTHER INFORMATION: /function= "endoglucanase" /EC_number= 3.2.1.4 /product= "BCE103 cellulase"
(ix) FEATURE:
   (A) NAME/KEY: CDS
   (B) LOCATION: 1..1404
(xi) SEQUENCE DESCRIPTION: SEQ ID NO: 1:
   ATG AAA AAG ATA ACT ACT ATT TTT GCC GTA TTG CTC ATG ACA TTG GCG        48
   Met Lys Lys Ile Thr Thr Ile Phe Ala Val Leu Leu Met Thr Leu Ala
   -26 -25                 -20                 -15
   TTG TTC AGT ATA GGA AAC ACG ACA GCG GCT GAT GAT TAT TCA GTT GTA        96
   Leu Phe Ser Ile Gly Asn Thr Thr Ala Ala Asp Asp Tyr Ser Val Val
   -10                  -5                   1               5
   GAG GAA CAT GGG CAA CTA AGT ATT AGT AAC GGT GAA TTA GTC AAT GAA 144
   Glu Glu His Gly Gln Leu Ser Ile Ser Asn Gly Glu Leu Val Asn Glu
   10 15 20
   CGA GGC GAA CAA GTT CAG TTA AAA GGG ATG AGT TCC CAT GGT TTG CAA 192
   Arg Gly Glu Gln Val Gln Leu Lys Gly Met Ser Ser His Gly Leu Gln
   25 30 35
   TGG TAC GGT CAA TTT GTA AAC TAT GAA AGC ATG AAA TGG CTA AGA GAT 240
   Trp Tyr Gly Gln Phe Val Asn Tyr Glu Ser Met Lys Trp Leu Arg Asp
   40 45 50
   GAT TGG GGA ATA ACT GTA TTC CGA GCA GCA ATG TAT ACC TCT TCA GGA 288
   Asp Trp Gly Ile Thr Val Phe Arg Ala Ala Met Tyr Thr Ser Ser Gly
   55 60 65 70
   GGA TAT ATT GAC GAT CCA TCA GTA AAG GAA AAA GTA AAA GAG ACT GTT 336
   Gly Tyr Ile Asp Asp Pro Ser Val Lys Glu Lys Val Lys Glu Thr Val
   75 80 85
   GAG GCT GCG ATA GAC CTT GGC ATA TAT GTG ATC ATT GAT TGG CAT ATC 384
   Glu Ala Ala Ile Asp Leu Gly Ile Tyr Val Ile Ile Asp Trp His Ile
   90 95 100
   CTT TCA GAC AAT GAC CCG AAT ATA TAT AAA GAA GAA GCG AAG GAT TTC 432
   Leu Ser Asp Asn Asp Pro Asn Ile Tyr Lys Glu Glu Ala Lys Asp Phe
   105 110 115
   TTT GAT GAA ATG TCA GAG TTG TAT GGA GAC TAT CCG AAT GTG ATA TAC 480
   Phe Asp Glu Met Ser Glu Leu Tyr Gly Asp Tyr Pro Asn Val Ile Tyr
   120 125 130
   GAA ATT GCA AAT GAA CCG AAT GGT AGT GAT GTT ACG TGG GAC AAT CAA 528
   Glu Ile Ala Asn Glu Pro Asn Gly Ser Asp Val Thr Trp Asp Asn Gln
   135 140 145 150
   ATA AAA CCG TAT GCA GAA GAA GTG ATT CCG GTT ATT CGT GAC AAT GAC 576
   Ile Lys Pro Tyr Ala Glu Glu Val Ile Pro Val Ile Arg Asp Asn Asp
   155 160 165
   CCT AAT AAC ATT GTT ATT GTA GGT ACA GGT ACA TGG AGT CAG GAT GTC 624
   Pro Asn Asn Ile Val Ile Val Gly Thr Gly Thr Trp Ser Gln Asp Val
   170 175 180
   CAT CAT GCA GCC GAT AAT CAG CTT GCA GAT CCT AAC GTC ATG TAT GCA 672
   His His Ala Ala Asp Asn Gln Leu Ala Asp Pro Asn Val Met Tyr Ala
   185 190 195
   TTT CAT TTT TAT GCA GGA ACA CAT GGA CAA AAT TTA CGA GAC CAA GTA 720
   Phe His Phe Tyr Ala Gly Thr His Gly Gln Asn Leu Arg Asp Gln Val
   200 205 210
   GAT TAT GCA TTA GAT CAA GGA GCA GCG ATA TTT GTT AGT GAA TGG GGG 768
   Asp Tyr Ala Leu Asp Gln Gly Ala Ala Ile Phe Val Ser Glu Trp Gly
   215 220 225 230
   ACA AGT GCA GCT ACA GGT GAT GGT GGT GTG TTT TTA GAT GAA GCA CAA 816
   Thr Ser Ala Ala Thr Gly Asp Gly Gly Val Phe Leu Asp Glu Ala Gln
   235 240 245
   GTG TGG ATT GAC TTT ATG GAT GAA AGA AAT TTA AGC TGG GCC AAC TGG 864
   Val Trp Ile Asp Phe Met Asp Glu Arg Asn Leu Ser Trp Ala Asn Trp
   250 255 260
   TCT CTA ACG CAT AAG GAT GAG TCA TCT GCA GCG TTA ATG CCA GGT GCA 912
   Ser Leu Thr His Lys Asp Glu Ser Ser Ala Ala Leu Met Pro Gly Ala
   265 270 275
   AAT CCA ACT GGT GGT TGG ACA GAG GCT GAA CTA TCT CCA TCT GGT ACA         960
   Asn Pro Thr Gly Gly Trp Thr Glu Ala Glu Leu Ser Pro Ser Gly Thr
       280                 285                 290
   TTT GTG AGG GAA AAA ATA AGA GAA TCA GCA TCT ATT CCG CCA AGC GAT        1008
   Phe Val Arg Glu Lys Ile Arg Glu Ser Ala Ser Ile Pro Pro Ser Asp
   295                 300                 305                 310
   CCA ACA CCG CCA TCT GAT CCA GGA GAA CCG GAT CCA GGA GAA CCG GAT        1056
   Pro Thr Pro Pro Ser Asp Pro Gly Glu Pro Asp Pro Gly Glu Pro Asp
                   315                 320                 325
   CCA ACG CCC CCA AGT GAT CCA GGA GAG TAT CCA GCA TGG GAT TCA AAT        1104
   Pro Thr Pro Pro Ser Asp Pro Gly Glu Tyr Pro Ala Trp Asp Ser Asn
               330                 335                 340
   CAA ATT TAC ACA AAT GAA ATT GTG TAT CAT AAC GGT CAG TTA TGG CAA        1152
   Gln Ile Tyr Thr Asn Glu Ile Val Tyr His Asn Gly Gln Leu Trp Gln
           345                 350                 355
   GCG AAA TGG TGG ACA CAA AAT CAA GAG CCA GGT GAC CCA TAC GGT CCG        1200
   Ala Lys Trp Trp Thr Gln Asn Gln Glu Pro Gly Asp Pro Tyr Gly Pro
       360                 365                 370
   TGG GAA CCA CTC AAA TCT GAC CCA GAT TCA GGA GAA CCG GAT CCA ACG        1248
   Trp Glu Pro Leu Lys Ser Asp Pro Asp Ser Gly Glu Pro Asp Pro Thr
   375                 380                 385                 390
   CCC CCA AGT GAT CCA GGA GAG TAT CCA GCA TGG GAT TCA AAT CAA ATT        1296
   Pro Pro Ser Asp Pro Gly Glu Tyr Pro Ala Trp Asp Ser Asn Gln Ile
                   395                 400                 405
   TAC ACA AAT GAA ATT GTG TAC CAT AAC GGC CAG CTA TGG CAA GCA AAA        1344
   Tyr Thr Asn Glu Ile Val Tyr His Asn Gly Gln Leu Trp Gln Ala Lys
               410                 415                 420
   TGG TGG ACA CAA AAT CAA GAG CCA GGT GAC CCA TAT GGT CCG TGG GAA        1392
   Trp Trp Thr Gln Asn Gln Glu Pro Gly Asp Pro Tyr Gly Pro Trp Glu
           425                 430                 435
   CCA CTC AAT TAA                                                        1404
   Pro Leu Asn
       440

### (2) INFORMATION FOR SEQ ID NO: 2:

(i) SEQUENCE CHARACTERISTICS:
   (A) LENGTH: 467 amino acids
   (B) TYPE: amino acid
   (D) TOPOLOGY: linear
(ii) MOLECULE TYPE: protein
(xi) SEQUENCE DESCRIPTION: SEQ ID NO: 2:
   Met Lys Lys Ile Thr Thr Ile Phe Ala Val Leu Leu Met Thr Leu Ala
   -26 -25                 -20                 -15
   Leu Phe Ser Ile Gly Asn Thr Thr Ala Ala Asp Asp Tyr Ser Val Val
   -10                 -5                  1               5
   Glu Glu His Gly Gln Leu Ser Ile Ser Asn Gly Glu Leu Val Asn Glu
               10                  15                  20
   Arg Gly Glu Gln Val Gln Leu Lys Gly Met Ser Ser His Gly Leu Gln
           25                  30                  35
   Trp Tyr Gly Gln Phe Val Asn Tyr Glu Ser Met Lys Trp Leu Arg Asp
   40 45 50
   Asp Trp Gly Ile Thr Val Phe Arg Ala Ala Met Tyr Thr Ser Ser Gly
   55 60 65 70
   Gly Tyr Ile Asp Asp Pro Ser Val Lys Glu Lys Val Lys Glu Thr Val
   75 80 85
   Glu Ala Ala Ile Asp Leu Gly Ile Tyr Val Ile Ile Asp Trp His Ile
   90 95 100
   Leu Ser Asp Asn Asp Pro Asn Ile Tyr Lys Glu Glu Ala Lys Asp Phe
   105 110 115
   Phe Asp Glu Met Ser Glu Leu Tyr Gly Asp Tyr Pro Asn Val Ile Tyr
   120 125 130
   Glu Ile Ala Asn Glu Pro Asn Gly Ser Asp Val Thr Trp Asp Asn Gln
   135 140 145 150
   Ile Lys Pro Tyr Ala Glu Glu Val Ile Pro Val Ile Arg Asp Asn Asp
   155 160 165
   Pro Asn Asn Ile Val Ile Val Gly Thr Gly Thr Trp Ser Gln Asp Val
   170 175 180
   His His Ala Ala Asp Asn Gln Leu Ala Asp Pro Asn Val Met Tyr Ala
   185 190 195
   Phe His Phe Tyr Ala Gly Thr His Gly Gln Asn Leu Arg Asp Gln Val
   200 205 210
   Asp Tyr Ala Leu Asp Gln Gly Ala Ala Ile Phe Val Ser Glu Trp Gly
   215 220 225 230
   Thr Ser Ala Ala Thr Gly Asp Gly Gly Val Phe Leu Asp Glu Ala Gln
   235 240 245
   Val Trp Ile Asp Phe Met Asp Glu Arg Asn Leu Ser Trp Ala Asn Trp
   250 255 260
   Ser Leu Thr His Lys Asp Glu Ser Ser Ala Ala Leu Met Pro Gly Ala
   265 270 275
   Asn Pro Thr Gly Gly Trp Thr Glu Ala Glu Leu Ser Pro Ser Gly Thr
   280 285 290
   Phe Val Arg Glu Lys Ile Arg Glu Ser Ala Ser Ile Pro Pro Ser Asp
   295 300 305 310
   Pro Thr Pro Pro Ser Asp Pro Gly Glu Pro Asp Pro Gly Glu Pro Asp
   315 320 325
   Pro Thr Pro Pro Ser Asp Pro Gly Glu Tyr Pro Ala Trp Asp Ser Asn
   330 335 340
   Gln Ile Tyr Thr Asn Glu Ile Val Tyr His Asn Gly Gln Leu Trp Gln
   345 350 355
   Ala Lys Trp Trp Thr Gln Asn Gln Glu Pro Gly Asp Pro Tyr Gly Pro
   360 365 370
   Trp Glu Pro Leu Lys Ser Asp Pro Asp Ser Gly Glu Pro Asp Pro Thr
   375 380 385 390
   Pro Pro Ser Asp Pro Gly Glu Tyr Pro Ala Trp Asp Ser Asn Gln Ile
                   395                 400                 405
   Tyr Thr Asn Glu Ile Val Tyr His Asn Gly Gln Leu Trp Gln Ala Lys
               410                 415                 420
   Trp Trp Thr Gln Asn Gln Glu Pro Gly Asp Pro Tyr Gly Pro Trp Glu
           425                 430                 435
   Pro Leu Asn
       440

## Description

### BACKGROUND OF THE INVENTION

### A. Technical field

The present invention relates to novel cellulase compositions. The invention further relates to novel cellulase compositions, preferably derived from *Bacillus sp.* The present invention further relates to the use of the novel cellulase in compositions recognized in the art as advantageously having cellulase added thereto, including, as an additive in a detergent composition, in the treatment of cellulose containing fabrics, in the treatment of pulp and paper and in the treatment of starch for the production of high fructose com-syrup or ethanol.

### B. State of the Art

Cellulases are enzymes which are capable of the hydrolysis of the 1,4 β-D-glucosidic linkages in celluloses. Cellulolytic enzymes have been traditionally divided into three major classes : endoglucanases, exoglucanases or cellobiohydrolases and β-glucosidases (Knowles, J. et al. (1987), TIBTECH 5, 255-261); and are known to be produced by a large number of bacteria, yeasts and fungi.

EMBL database accession number Z 33876.1 (Sanchez Torres et al) discloses the partial amino acid sequence of a cellulase from Bacillus strain N 186-1.

Primary among the applications that have been developed for the use of cellulolytic enzymes are those involving degrading (wood)cellulose pulp into sugars for (bio)ethanol production, textile treatments like 'stone washing' and 'biopolishing', and in detergent compositions. Thus, cellulases are known to be useful in detergent compositions for removing dirt, i.e., cleaning. For example, Great Britain Application Nos. 2,075,028, 2,095,275 and 2,094,826 illustrate improved cleaning performance when detergents incorporate cellulase. Additionally, Great Britain Application No. 1,358,599 illustrates the use of cellulase in detergents to reduce the harshness of cotton containing fabrics.

Another useful feature of cellulases in the treatment of textiles is their ability to recondition used fabrics by making their colors more vibrant. For example, repeated washing of cotton containing fabrics results in a greyish cast to the fabric which is believed to be due to disrupted and disordered fibrils, sometimes called "pills", caused by mechanical action. This greyish cast is particularly noticeable on colored fabrics. As a consequence, the ability of cellulase to remove the disordered top layer of the fiber and thus improve the overall appearance of the fabric has been of value.

Despite knowledge in the art related to many cellulase compositions having some or all of the above properties, there is a continued need for new cellulases having a varying spectrum of characteristics which are useful in, for example, treating textiles, as a component of detergent compositions, in the treatment of pulp and paper, and in the conversion of biomass. Applicants have discovered certain cellulases which have such a complement of characteristics and which are useful in such known applications of cellulase.

### SUMMARY OF THE INVENTION

It is an object of the present invention to provide a novel cellulase having beneficial properties for use in detergents treating textiles and pulp and paper manufacturing.

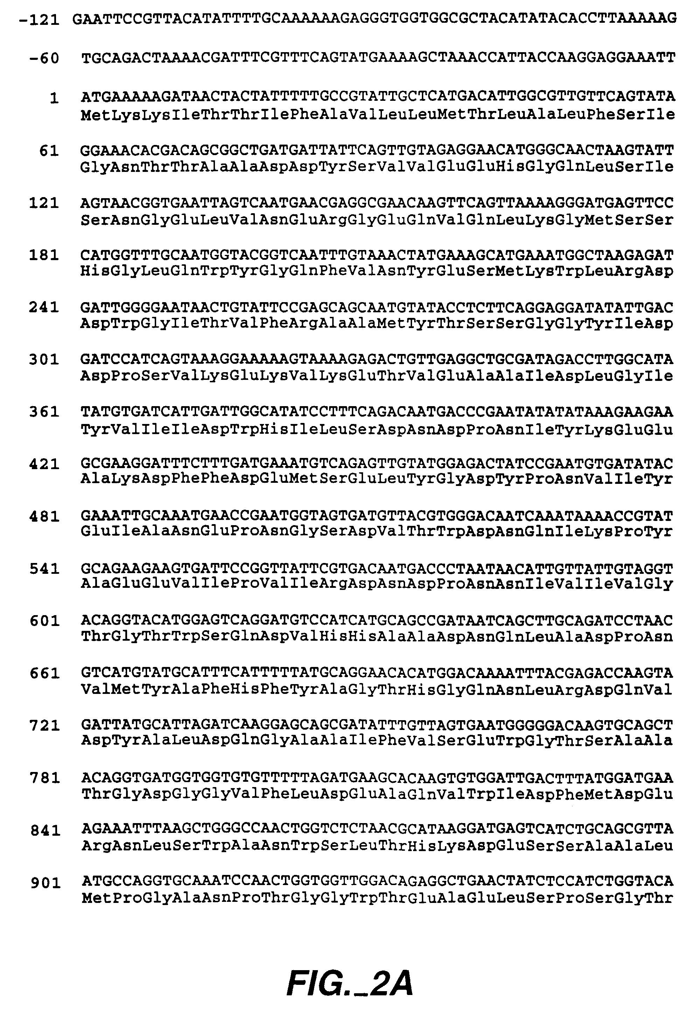

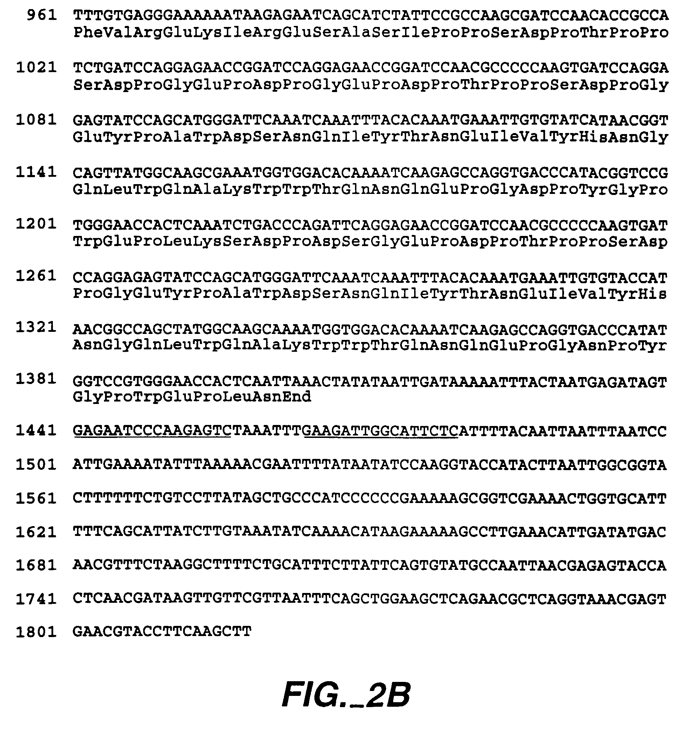

According to the present invention, a cellulase is obtainable from or derived from *Bacillus sp.* CBS 670 93. CBS 670.93 is deposited at the Centraalbureau voor Schimmelcultures (CBS), Baam, Netherlands under accession number CBS 670.93, on December 23, 1993 ("CBS 670.93"). The novel cellulase comprises an amino acid sequence according to Figure 2 (SEQ ID NO:2).

According to another embodiment, a composition is provided comprising DNA which encodes an amino acid sequence according to Figures 2 (SEQ ID NO:2).

According to yet another embodiment of the invention, a method of transforming a suitable microorganism with DNA encoding an amino acid sequence according to the invention is provided. Additionally, a microorganism transformed with DNA according to the invention is provided.

In an especially preferred embodiment of the present invention, the cellulase is a cellulase derived from *Bacillus sp.* CBS 670.93 having a calculated molecular weight of approximately 50 kD. The approximately 50 kD cellulase has a calculated isoelectric point of about 4 and a pH optimum on CMC of about 6-10 at 40°C and about 7 at 60°C.

### BRIEF DESCRIPTION OF THE FIGURES

Figure 1 shows the pH profile activity of an approximately 50 kD cellulase derived from CBS 670.93 at 40°C and 60°C.

Figure 2 shows the DNA sequence (SEQ ID. NO. 1) and deduced amino acid sequence (SEQ ID. NO. 2) for the 50 kD cellulase derived from CBS 670.93 with the leader peptide sequence underlined, which upon secretion is cleaved to yield the mature enzyme.

### DETAILED DESCRIPTION OF THE INVENTION

A cellulase is "obtainable from" *Bacillus* 670.93 if such cellulase has an amino acid sequence which corresponds to the amino acid sequence of a cellulase which may be obtained from that organism. Thus cellulase with an identical amino acid sequence to the 50 kD cellulase of the invention derived from a different *Bacillus* would be "obtainable from" *Bacillus* 670.93.

"Host cell" means a cell which has the capacity to act as a host and expression vehicle for a recombinant DNA vector according to the present invention In a preferred embodiment according to the present invention, "host cell" means the cells of *Bacillus.*

"DNA construct" or "DNA vector" means a nucleotide sequence which comprises one or more DNA fragments encoding any of the novel cellulases or cellulase derivatives described above.

In a preferred embodiment, the cellulase is obtainable from the Centraal Bureau voor Schimmelcultures, Baam, the Netherlands through microorganism deposition number CBS 670.93 (described in application PCT/EP94/04312), deposited under the Budapest Convention on December 23, 1993. As used herein, the deposited species will be referred to as CBS 670.93. In a more preferred embodiment, the cellulase of the invention is an approximately 50 kD cellulase (calculated on the basis of amino acid sequence of the mature protein) derived from CBS 670.93 (referred to herein as the "50 kD Cellulase"). The approximately 50 kD cellulase has a calculated pl for the mature protein of about 4 and a pH optimum on CMC of about 6-10 at 40°C and about 7 at 60°C.

The gene encoding the amino acid sequence of the approximately 50 kD cellulase was analyzed by comparison with the accessible sequence data in various libraries (GenBank, Swiss-Prot, EMBL and PIR) using the of CAOS/CAMM Center, University of Nijmegen, Holland. A search of databases for a comparison of the cellulase encoded by the DNA sequence of the present invention with cellulases encoded by published or known cellulase gene sequences revealed that the greatest amount of amino acid identity was found in the cellulase CelA of *Bacillus* sp. N-4 (Fukumori et al., J. Bacter., vol. 168, pp. 479-485 (1986)).

The approximately 50 kD cellulase was shown to be 89% identical in sequence and 92.5% similar in sequence using the TFastA program as described by Pearson & Lipman, Proc. Nat. Acad. Sci., vol. 85, pp. 2444-2448 (1988) to the closest published cellulase sequence. The TFastA Data Searching Program is commercially available in the Sequence Analysis Software Package Version 6.0 (Genetic Computer Group, Univ. Wisconsin Biotechnology Center, Madison, Wisconsin 53705).

The present invention also discloses a process for the production of the cellulase. In one embodiment, the cellulase may be produced by cultivating a suitable organism, e.g., *Bacillus sp.* CBS 670.93, under conditions so as to produce the cellulase. Preferably, such conditions include those generally suggested for the cultivation of *Bacillus* to maximize cellulase production and include the use of a cellulose derived substrate as an energy source in combination with necessary salts, ions and other well known ingredients. Generally, the medium used to cultivate the cells may be any conventional medium suitable for growing bacteria. The cells may be cultivated under aerobic conditions in a nutrient medium containing assimilable carbon and nitrogen together with other essential nutrients. Suitable carbon sources are carbohydrates such as sucrose, glucose and starch, or carbohydrate containing materials such as cereal grain, malt, rice and sorghum. The carbohydrate concentration incorporated in the medium may vary widely, e.g., up to 25% and down to 1-5%, but usually 8-10% will be suitable, the percentages being calculated as equivalents of glucose. The nitrogen source in the nutrient medium may be of inorganic and/or organic nature. Suitable inorganic nitrogen sources are nitrates and ammonium salts. Among the organic nitrogen sources used regularly in fermentation processes involving the cultivation of bacteria are soybean meal, cotton seed meal, peanut meal, casein, corn, corn steep liquor, yeast extract, urea and albumin. In addition, the nutrient medium should also contain standard trace substances.

The cellulase may be recovered from the medium by conventional procedures including separating the cells from the medium by centrifugation or filtration, if necessary after disruption of the cells, precipitating the proteinaceous components of the supernatant or filtrate by means of a salt, e.g., ammonium sulfate, followed by purification by a variety of chromatographic procedures, e.g., ion exchange chromatography, affinity chromatography or similar art recognized procedures. For the production of the alkaline cellulase according to the invention, it is preferred to cultivate under alkaline conditions using media containing a cellulose based energy source.

Preferably, the cellulase according to the present invention is produced utilizing genetic engineering techniques by transforming a suitable host cell with a gene encoding the cellulase and expressing under conditions appropriate for host cell growth and cellulase expression. As a first step, the chromosomal DNA may be obtained from the donor bacterial strain by the method of Saito and Miura (Saito & Miura, Biochim. Biophys. Acta., vol. 72, pp. 619 (1963)) or by a similar method. Restriction enzyme cleavage of the chromosomal DNA thus obtained gives DNA fragments containing the alkaline cellulase gene. For this purpose, any restriction enzyme may be used provided that it does not cleave the region of said gene. In the alternative, a restriction enzyme may be used which cleaves the gene, using however, a reduced enzyme concentration or incubation time to permit only partial digestion. A preferred restriction endonuclease is *Sau*3A. From the resulting digestion mixture, suitable fragments (2-6 kb) can be isolated and used to transform a suitable host cell with a DNA construct, e.g., with a DNA construct including the approximately 1.9 kb DNA fragment encoding the 50 kD cellulase according to the invention which has been ligated to a suitable vector sequence. The ligation mixture is then transformed into a suitable host.

The gene encoding the cellulase of the present invention can be cloned using λ-phage (expression) vectors and *E. coli host* cells. (Alternatively PCR cloning using consensus primers designed on conserved domains may be used). Applicants have discovered that transformation of the gene encoding the cellulase of the present invention and expression in *E. coli* results in an active protein. After a first cloning step in *E. coli,* a cellulase gene according to the present invention can be transferred to a more preferred industrial expression host such as *Bacillus* or *Streptomyces* species, a filamentous fungus such as *Aspergillus* or *Trichoderma,* or a yeast such as *Saccharomyces.* High level expression and secretion obtainable in these host organisms allows accumulation of the cellulase in the fermentation medium from which it can subsequently be recovered.

Preferably, the expression host cell comprises a *Bacillus* sp., more preferably *Bacillus licheniformis* or *Bacillus subtilis.* In an especially preferred embodiment, the transformation host is deleted for protease genes to ensure that the product cellulase is not subject to proteolysis in the fermentation broth or concentrates thereof. A preferred general transformation and expression protocol for protease deleted *Bacillus* strains is provided in Ferrari et al., U.S. Patent No. 5,264,366. Also preferably, the fermentation of the transformed *Bacillus* host is conducted at a pH of about 6.9. Transformation and expression in *Aspergillus* is described in, for example, Berka et al., U.S. Patent No. 5,364,770. A preferred promoter when the transformation host cell is *Bacillus* is the *apr*E promoter.

The instant approximately 50 kD cellulase derived from CBS 670.93 has been shown to be useful in buffer systems comprising glycine, ammonium acetate, borax and/or tris. This cellulase has also been found to be activated on CMC by the presence of magnesium and inhibited by the presence of calcium. A proportion of calcium to magnesium of about 750ppm : 250 ppm has also been found to result in an activity benefit.

According to the present invention, the cellulase compositions described above may be employed in detergent compositions according to art-recognized methods of utilizing cellulases in detergents. The excellent activity of the instant cellulase at alkaline pH should result in the present cellulase being especially useful in high pH detergents.

The invention will be explained in more detail in the following examples which are provided for illustrative purposes and should not to be construed as limitative of the invention.

### EXAMPLE 1

### Screening And Isolation of Cellulase From Alkaline Soil And Water Samples

Two methods were applied for the isolation of cellulase-producing microorganisms from alkaline soil and water samples. In one method, the soil and water samples were suspended in 0.85% saline solution and directly used in the carboxymethyl cellulose (CMC)-agar diffusion assay for detection of cellulase producing colonies. In a second method, the soil and water samples were enriched for cellulase containing strains by incubation in a cellulose containing liquid minimal medium or GAM-medium for 1 to 3 days at 40°C. Cultures that showed bacterial growth were analyzed for cellulase activity using the CMC-agar diffusion assay for detection of cellulase producing colonies. The CMC-agar diffusion assay and enrichment procedure utilized a minimal medium preparation at a pH of about 9.7 comprising 1% KNO₃, 0.1% yeast extract (Difco), 0.1% KH₂PO₄, 0.02% MgSO₄.7H₂O, 1% Na₂CO₃, 4% NaCI and 0.25% CMC (Sigma C-4888). For solidification 1.5% agar was added.

One of two procedures was used for the CMC-agar diffusion assay depending on whether colonies or liquid fractions were tested. For testing colonies, cell suspensions in 0.85% saline solution were plated on CMC-containing minimal medium. After incubation for 1 to 3 days at 40°C, the plates were replica plated and the parent plate was flooded with 0.1% Congo Red for 15 minutes. The plates were destained with 1M NaCI for 30 minutes. The strains that showed a clearing zone around the colony were isolated as potential cellulases producing microorganisms. Liquid fractions were assayed by pipetting 40 µl aliquots of enzyme solution or fermentation broth into wells punched out from a layer of 5 mm of minimal medium in a petri dish. After incubation for 16 hours at 40°C cellulase activity was detected by Congo Red / NaCl treatment. The diameter of the clearing zone is a measure for the CMCase activity.

Strains which showed clearing zones using either of the two screening methods were selected for growing up and isolation of cellulase. The colonies were fermented in 25 millilitre GAM-medium in 100 millilitre shake flasks in an Incubator Shaker (New Brunswick Scientific, Edison, NJ, USA), at 250 r.p.m. at 40°C for 72 hours. CMCase activity was determined in the culture broth at pH 9 and 40°C to verify the presence of cellulase in the fermentation broth. The complex medium (GAM) used for enzyme production consisted of Peptone (Difco) 0.5%, Yeast extract (Difco) 0.5%, Glucose. H₂O 1%, KH₂PO₄ 0.1%, MgSO₄.7H₂O 0.02%, Na₂CO₃ 1%, NaCI 4%. The pH was adjusted to 9.5 with 4M HCI after which 1% CMC was added.

Utilizing the method described above, a cellulase producing microorganism was isolated which was further characterized as small straight rods, occurring occaisonally in pairs and being motile. The terminal to sub-terminal spores were ellipsoidal with a clear swelling of the sporangium. Colonies on GAM-agar appeared as a creamy white, dull (i.e., not shiny) having an irregular surface with a filamentous margin. Based on 16S rRNA sequence analysis, the microorganism was classified as species of the genus *Bacillus.* The organism is referred to herein as CBS 670.93 and is deposited in the Centraal Bureau voor Schimmelcultures, Baam, The Netherlands under that accession number.

### EXAMPLE 2

### Isolation of DNA, Transformation and Expression of Cellulase

The alkaliphilic *Bacilli* strain CBS 670.93 was chosen as a donor strain for expression cloning in *E. coli.* Chromosomal DNA was isolated according to the method described by Saito & Miura, Biochim. Biophys. Acta., vol. 72, pp. 619-629 (1963).

The isolated chromosomal DNA is partially digested by the restriction enzyme *Sau*3A using serial diluted enzyme solutions, for one hour at 37°C using React Buffers (Gibco BRL Life Technologies, Gaithersburg, Md., USA) under conditions recommended by the supplier. The digested DNA is fractionated by agarose gel electrophoresis and suitable fractions (2-6 kb) are isolated from the gel using QlAquick Gel Extraction Kit according to the protocol described by the supplier (QIAGEN Inc., Chatsworth, Ca., USA).

The Sau3A fragments of the chromosomal DNA are used to construct genomic gene libraries in a *Bam*H1, digested CIAP treated ZAP Express vector according to the protocol described by the supplier (Stratagene Cloning Systems, La Jolla, Ca., USA). pBK-CMV phagmids, containing the cloned DNA inserts, were excised from the ZAP Express™ vector and transformed into *E. coli* strain XLOLR.

Recombinant clones are screened by agar diffusion as described by Wood et al., Meth. Enzym., vol. 160, pp. 59-74 (1988). Strains that showed clearing zones around the colony are isolated. The CMCase activity of the isolated recombinants is determined after fermentation for 48 hours in 4*YEP-medium consisting of Yeast Extract (Difco) 4%, peptone (Difco) 8%, lactose 0.2%, ampicillin 100µg/ml. The recombinant protein is purified (Example 3) and the amino acid sequence is determined (SEQ ID: NO 2).

Plasmid DNA of the cellulase producing recombinant is isolated using a QlAprep Plasmid Kit according to the protocol described by the supplier (QIAGEN Inc.). The plasmid contained an approximately 1.9 kb insert of chromosomal DNA. The nucleotide sequence of a fragment of 1933 bp is determined using a set of degenerated oligonucleotides derived from the N-terminal amino acid sequence as a primer to locate the gene on the 1.9 kb insert. The 1933 bp fragment contains an open reading frame of 1422 bp from which a protein of 467 amino acids can be deduced including a 26 amino acid leader sequence. The nucleotide sequence of the gene (SEQ. ID. NO:1) coding for said cellulase and the deduced amino acid sequence (SEQ ID NO:2) of the isolated single cellulase may then be determined and is illustrated in Figure 2.

### EXAMPLE 3

### Purification of Cellulase

The cellulase producing clones from Example 2 were grown on a complex medium (4*YEP) consisting of Yeast Extract (Difco) 4%, Peptone (Difco) 8%, lactose 0.2%, 100 µg/ml ampicillin). The fermentation broth was separated from the culture liquid by centrifugation (8000 rpm). The cellulase in the supernatant was precipitated with ammonium sulphate (65% saturation). The precipitate was dissolved in 25 mM phosphate buffer pH 7 + 5 mM EDTA until a conductivity of 7 mS/cm was achieved. This solution was applied to a Q-Sepharose FF (diameter 5 cm, length 10 cm) Anion Exchange column, after which the column was washed with 25 mM phosphate buffer pH 7 + 5 mM EDTA until an absorbency of 0.2 AU. A gradient of 0 to 0.5 M NaCI in 25 mM phosphate pH 7 was applied to the column in 80 minutes followed by a gradient from 0.5 to 1 M NaCI in 10 minutes. Elution took place in the first gradient. After elution the column was cleaned (upflow) with 1 M NaOH and equilibrated again with 25 mM phosphate pH 7 + 5 mM EDTA. Depending on the elution profile, the obtained cellulase had a purity of up to about 80%.

### EXAMPLE 4

### Properties of Cellulase According to the Invention

To determine the pH/temperature profile of the approximately 50 kD cellulase according to the invention, the activity of the cellulase was measured on CMC at various pH and temperature values. A solution comprising the approximately 50 kD cellulase was combined in a buffer in diluted with 10 mM phosphate buffer (pH 7). (pH was controlled by using buffer comprising a mixture of 100 ml 1 M phosphoric acid, 100 ml citric acid and 600 ml distilled water having the pH adjusted to 4, 5, 6, 7, 8, 9 or 10 using 4 M NaOH, after which the mixture is filled to 1 L using distilled water). The enzyme solution was diluted until 0.05 U/ml measured at pH 7 and 40°C. Each buffer system was tested to ascertain the actual pH after mixing 0.5 ml Buffer, 0.5 ml substrate (1% CMC) and 0.1 ml 10 mM phosphate buffer. Actual pH for the pH 4, 5, 6, 7, 8, 9 and 10 solutions was 4.2, 5.2, 6.2, 7, 8, 8.7 and 9.9, respectively.

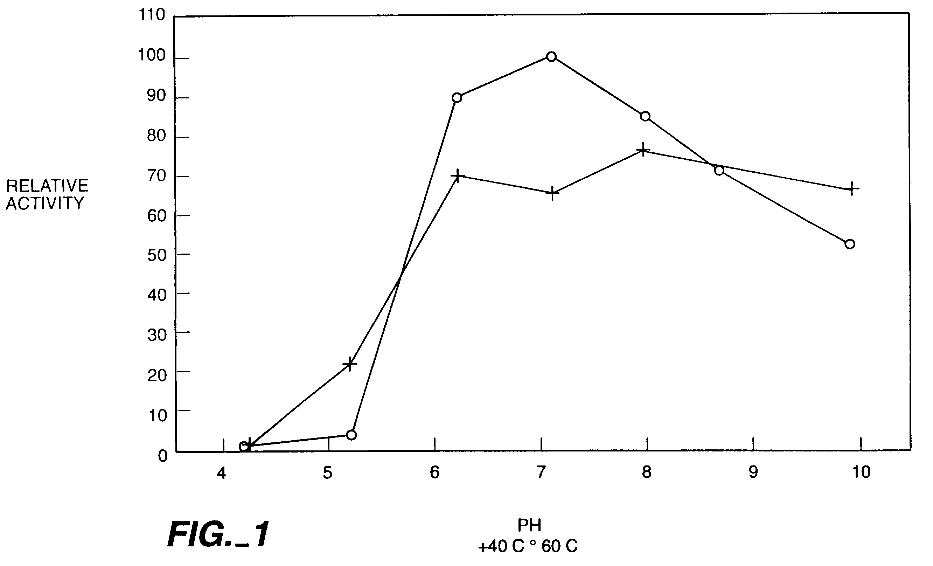

The results are illustrated in Figure 1 showing the excellent alkaline activity of the cellulase. The slope of the calibration curve is dependent on the pH of the enzyme substrate mixture for that reason two glucose standards at each pH are taken (500 mg glucose. H2)/100 ml 10 and 25 times diluted.

Cellulase activity may be assayed using a modified PAHBAH method (Lever M. Anal. Biochem. 1972, 47, 273-279 and Lever M. Anal. Biochem. 1977, 81, 21-27) as follows. The pH/temperature profiles may be determined using a fixed enzyme concentration which fits in the linear range of the dose response profile measured at pH 7 and 40°C. This enzyme concentration may be used for the measurement of the activities under all other determined conditions. A test tube is filled with 250 µl 2.5% CMC in 50 mM glycine buffer pH 9 (CMC-low viscosity is purchased from Sigma) and 250 µl aliquots of the 50 kD cellulase, diluted in the appropriate buffer. The test tube is incubated for 30 minutes at 40°C in a waterbath, whereafter 1.5 ml of a daily fresh prepared PAHBAH solution (1% PAHBAH in 100 ml 0.5 M NaOH with 100 ml bismuth solution (containing 48.5 g bismuth nitrate, 28.2 g potassium sodium tartrate and 12.0 g NaOH in 100 ml) is added. The mixture is heated at 70°C for 10 minutes, after which it is cooled on ice for 2 minutes. The absorption is measured at 410 nm. To eliminate the background absorbance of the enzyme samples a control experiment is executed as follows: a tube with substrate is incubated under the same conditions as the test tube. After the incubation 1.5 ml PAHBAH and the enzyme preparation is added (in this order). One unit (U) is defined as the amount of enzyme producing 1 µmol of glucose from CMC equivalent determined as reducing sugars per minute per gram product.

## Claims

1. A composition comprising a cellulase which comprises an amino acid sequence as set out in SEQ ID NO:2.

2. The composition of claim 1, wherein said cellulase is obtainable from *Bacillus sp.* CBS 670.93.

3. A composition comprising DNA which encodes the cellulase of claim 1.

4. An expression vector comprising the DNA of claim 3.

5. A host cell transformed with the DNA of claim 3.

6. A method of expressing a cellulase comprising:
(a) transforming a suitable microorganism with DNA encoding a cellulase of claim 1 or claim 2;
(b) preparing a fermentation broth containing said suitable microorganism under conditions suitable for expression of said DNA;
(c) maintaining said fermentation broth for a time and under conditions to permit the expression of a desired amount of said cellulase; and,
(d) collecting said fermentation broth which contains said cellulase.

7. A detergent composition comprising the cellulase of claim 1 or claim 2.

8. A method of treating textiles comprising contacting said textile with the cellulase of claim 1 or claim 2.

9. A method of treating cellulose based pulp comprising contacting said cellulose based pulp with the cellulase of claim 1 or claim 2.

## Patentansprüche

1. Zusammensetzung, umfassend eine Cellulase, die eine Aminosäuresequenz wie in Seq.-ID Nr. 2 dargelegt umfasst.

2. Zusammensetzung nach Anspruch 1, worin die Cellulase aus Bacillus sp. CBS 670.93 erhalten wird.

3. Zusammensetzung, umfassend DNA, die für die Cellulase nach Anspruch 1 kodiert.

4. Expressionsvektor, umfassend die DNA nach Anspruch 3.

5. Mit der DNA nach Anspruch 3 transformierte Wirtszelle.

6. Verfahren zum Exprimieren einer Cellulase, wobei das Verfahren umfasst:
(a) das Transformieren eines geeigneten Mikroorganismus mit DNA, die für eine Cellulase nach Anspruch 1 oder 2 kodiert;
(b) das Herstellen einer Fermentationsbrühe, die den geeigneten Mikroorganismus enthält, unter Bedingungen, die für die Expression der DNA geeignet sind;
(c) das Halten der Fermentationsbrühe für eine Zeit und unter Bedingungen, um die Expression einer gewünschten Menge der Cellulase zu ermöglichen; und
(d) das Sammeln der die Cellulase enthaltenden Fermentationsbrühe.

7. Detergenszusammensetzung, umfassend die Cellulase nach Anspruch 1 oder 2.

8. Verfahren zur Behandlung von Textilien, welches das Kontaktieren der Textilien mit der Cellulase nach Anspruch 1 oder 2 umfasst.

9. Verfahren zur Behandlung von Pulpe auf Cellulosebasis, welches das Kontaktieren der Pulpe auf Cellulosebasis mit der Cellulase nach Anspruch 1 oder 2 umfasst.

## Revendications

1. Composition comprenant une cellulase qui comprend une séquence d'acides aminés telle qu'indiquée à SEQ ID NO: 2.

2. Composition de la revendication 1 où ladite cellulase est obtenue de *Bacillus sp.* CBS 670.93.

3. Composition comprenant un ADN qui code pour la cellulase de la revendication 1.

4. Vecteur d'expression comprenant l'ADN de la revendication 3.

5. Cellule hôte transformée par l'ADN de la revendication 3.

6. Méthode d'expression d'une cellulase comprenant :
(a) la transformation d'un micro-organisme approprié avec un ADN codant pour une cellulase de la revendication 1 ou la revendication 2 ;
(b) la préparation d'un bouillon de fermentation contenant ledit micro-organisme approprié, dans des conditions appropriées à l'expression dudit ADN ;
(c) le maintien dudit bouillon de fermentation pendant un temps et dans des conditions pour permettre l'expression d'une quantité souhaitée de ladite cellulase ; et
(d) la collecte dudit bouillon de fermentation qui contient ladite cellulase.

7. Composition détergente comprenant la cellulase de la revendication 1 ou de la revendication 2.

8. Méthode de traitement de textiles comprenant la mise en contact dudit textile avec la cellulase de la revendication 1 ou de la revendication 2.

9. Méthode de traitement d'une pulpe à base de cellulose, comprenant la mise en contact de ladite pulpe à base de cellulose avec la cellulase de la revendication 1 ou de la revendication 2.
